(19) Europäisches Patentamt
European Patent Office
Office européen des brevets

(11) Veröffentlichungsnummer: **0 306 804**
**B1**

(12) **EUROPÄISCHE PATENTSCHRIFT**

(45) Veröffentlichungstag der Patentschrift: **06.02.91**

(51) Int. Cl.$^5$: **C 07 B 39/00,** C 07 C 63/04

(21) Anmeldenummer: **88114110.5**

(22) Anmeldetag: **30.08.88**

(54) **Herstellung von substituierten Benzotrichloriden.**

(30) Priorität: **11.09.87 DE 3730476**

(43) Veröffentlichungstag der Anmeldung:
**15.03.89 Patentblatt 89/11**

(45) Bekanntmachung des Hinweises auf die Patenterteilung:
**06.02.91 Patentblatt 91/06**

(84) Benannte Vertragsstaaten:
**AT BE CH DE ES FR GB IT LI NL SE**

(56) Entgegenhaltungen:
**GB-A- 898 307**

(73) Patentinhaber: **BAYER AG**
**D-5090 Leverkusen 1 Bayerwerk (DE)**

(72) Erfinder: **Döscher, Frank, Dr.**
**Sepp-Herberger-Strasse 32**
**D-4018 Langenfeld (DE)**
Erfinder: **Schnalke, Karl-Erwin, Dr.**
**Paul-Klee-Strasse 89**
**D-5090 Leverkusen (DE)**

**EP 0 306 804 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein Verfahren zur Herstellung von substituierten Benzotrichloriden, insbesondere von 2,4-Dichlor-5-fluorbenzotrichlorid, das über die Hydrolyse zum entsprechenden Benzoylchlorid führt und damit ein wichtiges Ausgangsprodukt für hochpotente antibakterielle Mittel wie das Ciprofloxacin darstellt.

Benzotrichloride stellen interessante Zwischenprodukte dar, da sie sich durch partielle oder vollständige Hydrolyse der Trichlormethylgruppe in die entsprechenden Benzal- oder Benzoylchloride oder durch Fluorierung in Benzotrifluoride umwandeln lassen.

Aus der GB—A—898 307 ist ein Verfahren zur Herstellung von im Kern chlorierten Benzolderivaten bekanntgeworden, wobei ein Chloratom spezifisch in die 4-Position eines monosubstituierten Benzolderivatives eingeführt wird. Als Katalysator wird ein üblicher, die aromatische Substitution begüngstigender Katalysator verwendet.

Die im erfindungsgemäßen Verfahren eingesetzten Ausgangsverbindungen zur Herstellung der substituierten Benzotrichloride entsprechen der Formel (I),

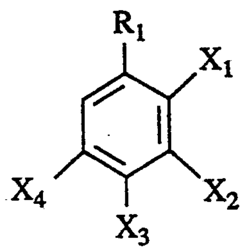

(I)

worin

$R_1$ für Methyl, Methoxy oder Thiomethyl steht, und

$X_1$, $X_2$, $X_3$ und $X_4$ gleich oder verschieden sind und Wasserstoff und Halogen bedeuten und $X_3$ auch —COCl und —NCO sein kann.

Die Chlorierung der Methylgruppe verläuft bekanntermaßen nach einem radikalischen Mechanismus. Dabei ist der Einsatz von Radikalbildnern, z.B. organischen Peroxiden, UV-Licht oder hohen Temperaturen (>180°C) notwendig. Die Verwendung von organischen Peroxiden bietet in bezug auf Applikation und Auswahl der Halbwertszeit verfahrentechnische Vorteile. Nachteilig ist, daß manche Peroxide, wie z.B. Dibenzoylperoxid, als wäßrige Paste (Dephlegmatisierung) geliefert werden. Die Chlorierung der Methylgruppe zur gewünschten Verbindung läßt sich aber im allgemeinen nur durchführen, wenn man die Peroxide dem Ansatz in wasserfreier, gelöster Form kontinuierlich zudosiert. Als gute Lösemittel für die organischen Peroxide dienen Methylenchlorid oder Tetrachlorkohlenstoff, die jedoch bei den Chlorierungstemperaturen (>100°C) sofort verdampfen und damit das Reaktionsabgas und das Abwasser stark belasten.

Gegenstand der Erfindung ist nun ein sowohl diskontinuierliches als auch kontinuierliches Verfahren zur Herstellung von substituierten Benzotrichloriden durch Seitenkettenchlorierung im Substituenten $R_1$ von Verbindungen der Formel (I),

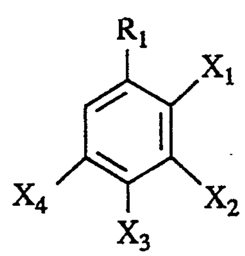

(I)

in der

$R_1$ für Methyl, Methoxy oder Thiomethyl steht,

$X_1$, $X_2$, $X_3$ und $X_4$ gleich oder verschieden sind und Wasserstoff und Halogen bedeuten und $X_3$ auch

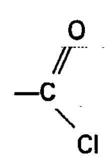

oder —NCO sein kann, das dadurch gekennzeichnet ist, daß man in einer Anlaufphase Chlorierungskatalysatoren in Verbindungen der Formel (I) löst, diese Lösung zusammen mit Chlor in ein Reaktionsgefäß eindosiert, in dem Verbindungen der Formel (I) zur Chlorierung vorgelegt sind und ständig chloriert und daß man nach der Anlaufphase aus dem Reaktionsgefäß teilchlorierte bzw. endchlorierte Verbindungen, die sich von der Formel (I) ableiten, abzieht, in diesen Verbindungen frischen Chlorierungskatalysator löst

und diese Katalysatorlösung zusammen mit Chlor dem Reaktionsgefäß zuführt.

Beispielsweise wird so vorgegangen, maß man in einem geeigneten Emailkessel 600 bis 1000 kg 2,4-Dibrom-5-fluortoluol vorlegt und auf bis 150°C erhitzt, in einem 100 l Emailkessel 60 bis 100 kg 2,4-Dibrom-5-fluortoluol — im späteren Verlauf 60 bis 100 kg der teilchlorierten Substanz — vorlegt, 2 bis 6 kg eines peroxidischen Katalysators einträgt und bei 30 bis 60°C löst,

die Katalysatorlösung mit etwa 3 bis 6 l/h in den Chlorierungsansatz eindosiert,

in das Chlorierungsprodukt 2,4-Dichlor-5-fluorbenzotrichlorid überführt und

letzteres gegebenenfalls durch Hydrolyse in 2,4-Dichlor-5-fluorbenzoylchlorid umwandelt.

Als Katalysator wird bevorzugt Di-lauroylperoxid verwendet. Es können jedoch auch andere Peroxid-Katalysatoren verwendet werden. Für die Auswahl ist maßgebend ihre Löslichkeit in dem anchlorierten Reaktionsgemisch und ihre Halbwertszeit, die sich nach der Chlorierungstempertur richtet. Infrage hierfür kommen z.B. Di-octanoylperoxid, Bis-(2-methylbenzoyl)-peroxid, t-Butyl-peroxipivaloat.

Vorteile:

1. Keine Verwendung von wasserhaltiger Katalysatorsubstanz, so daß eine Trocknung entfällt.

2. Keine Verwendung von leichtsiedenden Chlorkohlenwasserstoffen wie Tetrachlorkohlenstoff und Methylenchlorid, so daß die aufwendige Abtrennung aus dem Prozeßabwasser und der Abluft entfällt.

3. Einfachere Reinigungsmöglichkeiten für das wiedergewonnene Brom, da die mögliche Azeotropbildung mit dem Lösungsmittel entfällt.

Bevorzugt werden für erfindungsgemäße Verfahren Verbindungen der Formel (I) verwendet, in denen $X_4$ Fluor und $X_1$ Chlor oder Brom und $X_3$ Cl, Br, NCO oder

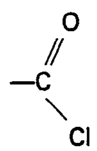

bedeuten.

Besonders bevorzugt werden für das erfindungsgemäße Verfahren Verbindungen der Formel (I) verwendet, in denen $R_1$ Methyl bedeutet.

Insbesondere werden für das Verfahren Verbindungen der Formel (I) verwendet, in denen $R_1$ Methyl, $X_4$ Fluor und $X_1$ und $X_3$ beide Brom bedeuten.

Beispiel 1

Im 500 l Emailkessel werden 600 kg der zu chlorierenden Verbindung vorgelegt und auf 130°C aufgeheizt.

In einem 100 l Emailkessel werden 60 kg (bzw. im späteren Verlauf 60 kg der teilchlorierten Substanz) vorgelegt;

3 kg Di-lauroylperoxid werden eingetragen und bei 30 bis 40°C gelöst. Diese Katalysatorlösung wird mit ca. 3 l/h in den Chlorierungszeit eindosiert. Während einer Chlorierungszeit von etwa 50 h werden ca. 1000 kg Chlor und 12 bis 15 kg Peroxid-Katalysator verbraucht. Der Gehalt des Rohproduktes (715 kg) beträgt ca. 98% nach GC.

Nach diesem Verfahren werden pro Tonne 2,4-Dibrom-5-fluortoluol ca. 20 kg Di-lauroylperoxid benötigt (Basis: 0,5 $m^3$ Kessel); weitere Hilfsstoffe sind nicht erforderlich.

**Beispiel 2**

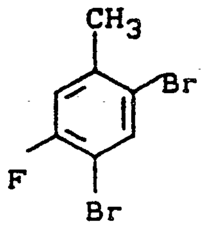

618 g 2,4-Dibrom-5-fluor-toluol

6,9 g Di-lauroylperoxid (~0,5 g/h)

13 h Reaktionszeit unter Chloreinleitung im Überschuß

2100 g Endprodukt (2,4-Dichlor-5-fluor-benzotrichlorid)

97,6 % Ausbeute

**Beispiel 3**

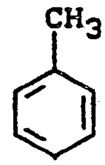

387 g Toluol

15 g Di-lauroylperoxid (~1,0 g/h)

14 h Reaktionszeit unter Chloreinleitung im Überschuß

754 g Endprodukt (Benzotrichlorid)

94,7 % Ausbeute

**Beispiel 4**

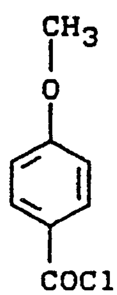

900 g p-Methoxibenzoylchlorid

15 g Di-lauroylperoxid (~ 0,75 g/h)

20 h Reaktionszeit unter Chloreinleitung im Überschuß

1373 g Endprodukt (Trichlormethoxibenzoylchlorid)

93 % Ausbeute

**Beispiel 5**

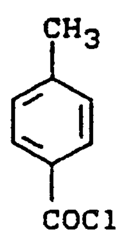

4275 g 4-Methyl-benzoylchlorid

44 g Di-lauroylperoxid (~ 1 g/h)

40 h Reaktionszeit unter Chloreinleitung im Überschuß

6647 g Endprodukt (4-Trichlormethylbenzoylchlorid)

90 % Ausbeute

<u>Beispiel 6</u>

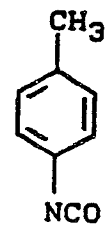

1200 g p-Tolylisocyanat

12 g Di-lauroylperoxid (- 1 g/h)

12 h Reaktionszeit unter Chloreinleitung im Überschuß

1970 g Endprodukt (92 %ig) (p-Trichlormethylphenylisocyanat)

95 % Ausbeute

Nach der gleichen Methode ist auch

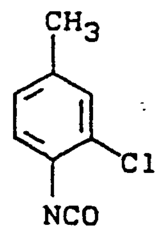

chlorierbar (2-Chlor-4-trichlormethylphenylisocyanat).

**Patentansprüche**

1. Verfahren zur Herstellung von substituierten Benzotrichloriden durch Seitenkettenchlorierung im Substituenten $R_1$ von Verbindungen der Formel (I)

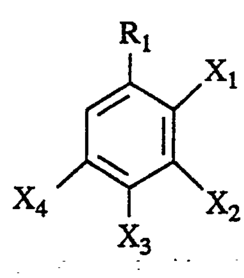

(I)

in der

$R_1$ für Methyl, Methoxy oder Thiomethyl steht,

$X_1$, $X_2$, $X_3$ und $X_4$ gleich oder verschieden sind und Wasserstoff und Halogen bedeuten und $X_3$ auch

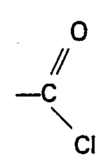

oder —NCO sein kann, dadurch gekennzeichnet, daß man in einer Anlaufphase Chlorierungskatalysatoren, bein denen es sich um im anchlorierten Reaktionsgemisch lösliche Peroxid-Katalysatoren handelt, in Verbindungen der Formel (I) löst, diese Lösung zusammen mit Chlor in ein Reaktionsgefäß eindosiert, in dem Verbindungen der Formel (I) zur Chlorierung vorgelegt sind und ständig chloriert und daß man nach der Anlaufphase aus dem Reaktionsgefäß teilchlorierte bzw. endchlorierte Verbindungen, die sich von der Formel (I) ableiten, abzieht, in diesen Verbindungen frischen Chlorierungskatalysator löst und diese Katalysatorlösung zusammen mit Chlor dem Reaktionsgefäß zuführt.

2. Verfahren nach Anspruch 1, dadurch gekennzeichnet, daß man solche Verbindungen der Formel (I) verwendet, in denen $X_4$ Fluor und $X_1$ Cl oder Br und $X_3$ Cl, Br,

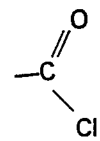

oder —NCO bedeuten.

3. Verfahren nach Anspruch 1, dadurch gekennzeichnet, daß man Verbindungen der Formel (I) verwendet, in denen $R_1$ Methyl bedeutet.

4. Verfahren nach Anspruch 1, dadurch gekennzeichnet, daß man Verbindungen der Formel (I) verwendet, in denen $R_1$ Methyl, $X_4$ Fluor und $X_1$ und $X_3$ beide Brom bedeuten.

5. Verfahren nach Anspruch 3, dadurch gekennzeichnet, daß man in einem geeigneten Emailkessel 600 bis 1000 kg 2,4-Dibrom-5-fluortoluol vorlegt und auf 100 bis 150°C erhitzt, in einem 100 l Emailkessel 60 bis 100 kg 2,4-Dibrom-5-fluortoluol — im späteren Verlauf 60 bis 100 kg der teilchlorierten Substanz — vorlegt, 2 bis 6 kg eines peroxidischen Katalysators einträgt und bei 30 bis 60°C löst, die Katalysatorlösung mit etwa 3 bis 6 l/h in den Chlorierungsansatz eindosiert, in das Chlorierungsprodukt 2,4-Dichlor-5-fluorbenzotrichlorid überführt und letzteres gegebenenfalls durch Hydrolyse in 2,4-Dichlor-5-fluorbenzoylchlorid umwandelt.

6. Verfahren nach Anspruch 4, dadurch gekennzeichnet, daß man als Katalysator Di-lauroylperoxid verwendet.

**Revendications**

1. Procédé pour la fabrication de trichlorures de benzényle substitutés par chloration en chaîne latérale dans le substituant $R_1$ de composés de formule (I)

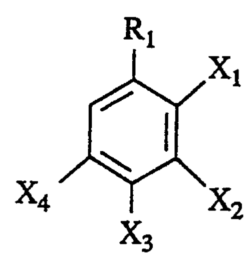

(I)

dans laquelle

$R_1$ représente un groupe méthyle, méthoxy ou thiométhyle, et

$X_1$, $X_2$, $X_3$ et $X_4$ sont identiques ou différents et représentent un atome d'hydrogène ou un atome d'halogène et

$X_3$ peut également représenter

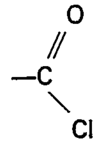

ou —NCO, caractérisé en ce que, dans une phase de démarrage, on dissout dans des composés de formule (I) des catalyseurs de chloration, en l'occurrence des catalyseurs peroxydiques solubles dans le mélange réactionnel chloré, qu'on ajoute cette solution par addition dosée en même temps que la chlore dans un réacteur dans lequel des composés de formule (I) sont présentés à la chloration et sont chlorés en continu, et qu'après la phase de démarrage on extrait du réacteur des composés partiellement chlorés ou complètement chlorés dérivant de la formule (I), qu'on dissout dans ces composés du catalyseur de chloration frais et qu'on amène cette solution de catalyseur avec le chlore dans le réacteur.

2. Procédé selon la réaction 1, caractérisé en ce que l'on utilise des composés de formule (I), dans lesquels $X_4$ représente le fluor, $X_1$ représente le chlore ou le brome et $X_3$ représente

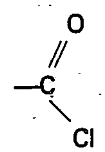

ou —NCO.

3. Procédé selon la revendication 1 caractérisé en ce que l'on utilise des composés de formule (I) dans lesquels $R_1$ représente un groupe méthyle.

4. Procédé selon la revendication 1, caractérisé en ce que l'on utilise des composés de formule (I) dans lesquels $R_1$ représente un groupe méthyle, $X_4$ représente le fluor et $X_1$ et $X_3$ représente tous deux brome.

5. Procédé selon la revendication 3 caractérisé en ce qu'on introduit dans une cuve en émail appropriée 600 à 1000 kg de 2,4-dibromo-5-fluorotoluène et qu'on chauffe à 100—150°C, qu'on introduit dans une cuve en émail de 100 l 60 à 100 kg de 2,4-dibromo-5-fluorotoluène, dans le suite du déroulement, 60 à 100 kg de substance partiellement chlorée, qu'on introduit 2 à 6 kg d'un catalyseur peroxydique et qu'on le dissout à une température de 30 à 60°C, qu'on ajoute par addition dosée à raison de 6 l/h la solution de catalyseur dans la préparation de chloration, on effectue la transformation en produit de chloration, trichlorure de 2,4-dichloro-5-fluorobenzényle et on transforme éventuellement ce dernier par hydrolyse en 2,4-dichloro-5-fluorobenzényle.

6. Procédé selon la revendication 4, caractérisé en ce que l'on utilise comme catalyseur du peroxyde de dilayroyle.

**Claims**

1. Process for the preparation of substituted benzotrichlorides by side-chain chlorination in the substituent $R_1$ of compounds of the formula (I)

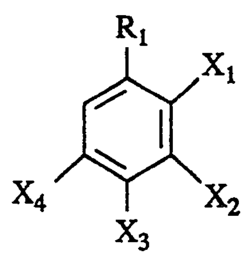

(I)

in which

$R_1$ represents methyl, methoxy or thiomethyl,

$X_1$, $X_2$, $X_3$ and $X_4$ are identical or different and denote hydrogen and halogen and $X_3$ may alternatively be

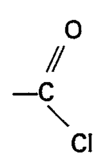

or —NCO, characterized in that, in an initial phase, chlorination catalysts, these being peroxide catalysts soluble in the partially chlorinated reaction mixture, are dissolved in compounds of the formula (I), this solution, together with chlorine, is metered into a reaction vessel into which compounds of the formula (I) have been initially introduced for chlorination, and the chlorination is carried out continuously, and in that, after the initial phase, partially or fully chlorinated compounds derived from the formula (I) are removed from the reaction vessel, fresh chlorination catalyst is dissolved in these compounds, and this catalyst solution, together with chlorine, is fed to the reaction vessel.

2. Process according to Claim 1, characterized in that those compounds of the formula (I) in which $X_4$ denotes fluorine and $X_1$ denotes Cl or Br and $X_3$ denotes Cl, Br,

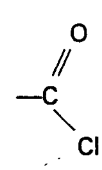

or —NCO are used.

3. Process according to Claim 1, characterized in that compounds of the formula (I) in which $R_1$ denotes methyl are used.

4. Process according to Claim 1, characterized in that compounds of the formula (I) in which $R_1$ denotes methyl, $X_4$ denotes fluorine, and $X_1$ and $X_3$ both denote bromine are used.

5. Process according to Claim 3, characterized in that 600 to 1,000 kg of 2,4-dibromo-5-fluorotoluene are initially introduced into a suitable enamel kettle and heated to 100 to 150°C, 60 to 100 kg of 2,4-dibromo-5-fluorotoluene — later in the procedure 60 to 100 kg of the partially chlorinated substance — are initially introduced into a 100 l enamel kettle, 2 to 6 kg of a peroxide-type catalyst are added and dissolved at 30 to 60°C, the catalyst solution is metered into the chlorination batch at a rate of approximately 3 to 6 l/h, the mixture is converted into the chlorination product 2,4-dichloro-5-fluorobenzotrichoride, and the latter is converted into 2,4-dichloro-5-fluorobenzoyl chloride, if appropriate by hydrolysis.

6. Process according to Claim 4, characterized in that the catalyst used is d-lauroyl peroxide.